# EUROPÄISCHE PATENTSCHRIFT

(11) **EP 3 122 283 B1**
(45) Veröffentlichungstag und Bekanntmachung des Hinweises auf die Patenterteilung: **14.02.2018**
(21) Anmeldenummer: 15714779.4
(22) Anmeldetag: 27.03.2015
(51) Int. Cl.: A61C 9/00, A61B 1/00, A61B 5/00, G01B 11/24

(54) **SCANVORRICHTUNG**
SCANNING DEVICE
DISPOSITIF DE BALAYAGE

(30) Priorität: 27.03.2014 DE 102014205784
(43) Veröffentlichungstag der Anmeldung: 01.02.2017
(73) Patentinhaber: Sirona Dental Systems GmbH, 64625 Bensheim (DE)
(72) Erfinder: BERNER, Markus, CH-8180 Bülach (CH); THIEL, Frank, 64372 Ober-Ramstadt (DE)
(74) Vertreter: Sommer, Peter
(86) Internationale Anmeldenummer: PCT/EP2015/056694
(87) Internationale Veröffentlichungsnummer: WO 2015/144875

(56) Entgegenhaltungen:
- DE-A1- 4 034 007
- US-A- 5 372 502
- US-A- 5 745 165
- US-A1- 2002 058 229
- US-A1- 2004 254 476
- US-B1- 6 386 867

## Beschreibung

Die Erfindung betrifft einen Scanvorrichtung. Insbesondere betrifft die Erfindung einen Ganzkiefer-Scanner.

Es existiert der Wunsch, digitale dreidimensionale Daten bzw. 3D-Daten der Zähne bzw. des Kiefers von Patienten zu erhalten, ohne einen Silikonabdruck erstellen zu müssen.

Im Stand der Technik gibt es Scanner, wie "CEREC OmniCam", die in den Mund des Patienten eingeführt werden und dann bei einer Aufnahme nur einen kleinen Ausschnitt eines Zahnbogens dreidimensional vermessen können. Der Scanner muss dann manuell im Mundraum herumgeführt werden, um dabei eine Vielzahl von Einzelaufnahmen zu machen und dann zu einem Gesamtbild zusammenzufügen. Der Anwender trägt dabei dafür die Verantwortung, dass der gesamte Kiefer vollständig und lückenlos erfasst wird. Das erfolgt über eine visuelle Kontrolle des Resultats und dann eine manuelle Positionierung des Scanners an eine noch zu erfassende Stelle.

Bei einem solchen Scannen gibt es Probleme in Bezug auf die Präzision des Scannens infolge von Fehlerfortpflanzung bei der Zusammenführung zu einem Gesamtbild. Weiterhin ist ein manuelles Herumführen des Scanners im Mundraum zeitraubend und auch nicht so einfach, dass ein Anwender ohne vorherige Schulung tätig werden kann. Auch die visuelle Kontrolle der Vollständigkeit und das darauffolgende manuelle Positionieren des Geräts an der zu vermessenden Stelle sind nicht einfach, so dass die Gefahr besteht, dass die Daten unvollständig sind.

Es gibt weiterhin Scanner wie "iTray", wobei ein Patient auf eine flexible Masse beissen muss, die dann direkt im Mund vermessen wird.

Bei dieser Art von Scannen ist zum einen die Handhabung einer flexiblen Masse problematisch. Zudem ist eine Vielzahl von Sensoren nötig.

DE 10 2008 047 816 B4 zeigt einen 3D-Scanner zum Abtasten von Zähnen mit Träger und Griffteil, wobei auf dem Träger Projektoren und mehrere Kameras vorgesehen sind. Zudem ist ein Kalibrieren der Kameras angegeben und ist ein "Registrieren" oder "Matchen", ein Zusammenfügen von Einzelaufnahmen, beschrieben.

WO 2013/121605 A1 und DE 11 2012 005 886 T5 zeigen eine Vorrichtung zur Zahnreihenaufnahme in Mundhöhlen, wobei mehrere Kameras zur (gleichzeitigen) Aufnahme von Zähnen vorgesehen sind.

US 2002/0058229 A1 zeigt das Vorsehen von transparentem Material an einem Mundstück einer Vorrichtung zur Abbildung von Zähnen.

US 6 386 867 B1 zeigt ein Verfahren zum Scannen von Zähnen in Einzelscans mit einem Abfragen, ob alles gescannt ist, und darauffolgendem Erzeugen eines 3D-Modells.

Auch US 6 821 116 B1 zeigt ein Verfahren zum Scannen von Zähnen.

DE 10 2009 038 588 A1 zeigt ein Verfahren zur Ermittlung eines Gesamtdatensatzes eines zu messenden Objekts aus Einzeldatensätzen (mit Korrektur).

DE 38 10 455 A1 zeigt ein Verfahren und eine Vorrichtung zur berührungsfreien räumlichen Erfassung eines unregelmässigen Körpers, wobei eine Antriebseinheit zum Antreiben von Aufnahmeeinheiten von einer Auswerteeinheit steuerbar ist.

Das nächstliegende Dokument DE 42 18 219 C2 zeigt eine Vorrichtung zum berührungslosen Vermessen von beispielsweise zahntechnischen Objekten, wobei eine Antriebseinrichtung zum Antreiben einer Erfassungseinrichtung zum Durchführen eines Einzelscans und eine Steuereinrichtung zum Steuern der Antriebseinrichtung zum Bewegen der Erfassungseinrichtung zu einer bestimmten Stelle vorgesehen sind.

Es ist die Aufgabe der vorliegenden Erfindung, einen Ganzkiefer-Scanner und ein zugehöriges Verfahren zur Verfügung zu stellen, wobei das Scannen auf einfache Weise erfolgt und 3D-Daten eines gesamten Kiefers mit hoher Präzision erhalten werden.

Die Aufgabe wird gelöst durch eine Scanvorrichtung, wie sie im Anspruch 1 angegeben ist, und durch ein Scanverfahren, wie es im Anspruch 16 angegeben ist. Die jeweils abhängigen Ansprüche 2 bis 15 und 17 bis 23 zeigen vorteilhafte Weiterbildungen der Scanvorrichtung des Anspruchs 1 bzw. des Scanverfahrens des Anspruchs 16.

Es wird eine Scanvorrichtung mit einem von einer Bedienperson zu haltenden Griff und einem Mundstück zur Verfügung gestellt, das in den Mund eines Patienten einzuführen ist, wobei die Scanvorrichtung eine im Mundstück vorgesehene Erfassungseinrichtung und eine Scaneinrichtung aufweist, wobei die Erfassungseinrichtung in Verbindung mit der Scaneinrichtung bei in den Mund eines Patienten eingeführtem Mundstück bei einer Aufnahme jeweils einen Ausschnitt eines Zahnbogens im Mund scannt und dreidimensional vermisst, wobei viele Einzelscans von verschiedenen Ausschnitten des Zahnbogens aufgenommen und zu einem Gesamtbild zusammengefügt werden.

Die Scanvorrichtung weist weiterhin folgendes auf: eine Antriebseinrichtung, die die Erfassungseinrichtung derart antreibt, dass sie sich von einem Ausschnitt zu einem nächsten Ausschnitt des Zahnbogens bewegt, um einen jeweiligen Einzelscan durchzuführen, und eine Steuereinrichtung, die die Antriebseinrichtung derart steuert, dass sie die Erfassungseinrichtung zu einer gewünschten Stelle des Zahnbogens bewegt, wobei die Erfassungseinrichtung bevorzugt wenigstens einen Spiegel aufweist, der im Mundstück mittels der Antriebseinrichtung motorisch angetrieben so bewegt wird, dass mit ihm ein Scanbereich an eine gewünschte Stelle des Zahnbogens abgelenkt wird. Erfindungsgemäß ist für das Mundstück eine Umhüllung vorgesehen, die wenigstens teilweise transparent ist, wobei auf die Umhüllung des Mundstücks bzw. das transparente Mundstück präzise Kalibrierungsmarken aufgebracht sind, die zusammen mit einem jeweiligen Einzelscan gescannt werden.

Die Scanvorrichtung mit bewegbarer Erfassungseinrichtung macht es auf vorteilhafte Weise möglich, einen gesamten Kiefer zu scannen, ohne dass die gesamte Scanvorrichtung von einer Bedienperson im Mund eines Patienten neu positioniert werden muss.

Bevorzugt hat der wenigstens eine Spiegel wenigstens drei Freiheitsgrade, wodurch mittels der Scaneinrichtung im Mund eines Patienten lingual und/oder okklusal und/oder bukkal gescannt werden kann.

Vorzugsweise weist die Steuereinrichtung auch eine Berechnungs- und Auswerteeinrichtung zum Zusammenfügen der Einzelscans zum Erhalten der vollständigen Kieferdaten unter Berücksichtigung der gescannten Kalibrierungsmarken und somit der Position der Spiegel, zur Analyse der erhaltenen Kieferdaten zur Feststellung einer Vollständigkeit der Kieferdaten und zur Vervollständigung der Kieferdaten auf.

Das Zusammenfügen der Einzelscans erfolgt beispielsweise mittels eines iterativen Verfahrens zur schrittweisen Annäherung zweier Punktwolken, das auch ICP-(= Iterative Closest Point)Verfahren genannt wird. Die ungefähre Position der Spiegel kann zur Plausibilitätsprüfung herangezogen werden.

Durch Kenntnis der Lage der Kalibrierungsmarken auf dem Mundstück kann das Gesamtresultat korrigiert werden. Es kann festgestellt werden, ob die Messung bereits vollständig ist oder nicht, das heisst, ob die Oberfläche eines Zahnbogens bereits vollständig erfasst wurde oder ob es noch Leerstellen in der gemessenen Oberfläche gibt. Eine Scanposition für einen Einzelscan wird dann automatisch an die Stelle von solchen Leerstellen gelegt. Falls nötig werden solche Stellen unter mehreren Blickwinkeln gemessen. Das durch den Einzelscan erhaltene Messergebnis wird dann mit den übrigen Einzelscans zusammengefügt.

Die Scaneinrichtung ist vorzugsweise ein 3D-Scanner, der über einen grossen Tiefenbereich, wie beispielsweise 100 mm, scannen kann. Durch eine solche Scaneinrichtung können gute Bilder aufgenommen werden.

Das motorische Antreiben erlaubt es auf vorteilhafte Weise, sowohl die Lage als auch die Blickrichtung des Einzelscans frei zu wählen und zu positionieren. Durch das motorische Antreiben von Spiegeln ist ein manuelles Bewegen der Scanvorrichtung im Mund eines Patienten nicht mehr nötig, was sowohl für den Patienten als auch eine die Scanvorrichtung bedienende Person eine grosse Erleichterung mit sich bringt. Auch kann durch das motorische Antreiben eine Genauigkeit beim Scannen verbessert werden.

Als bewegbare Erfassungseinrichtung kann ein Spiegel vorgesehen sein, wobei auch die Scaneinrichtung bewegbar angeordnet ist. Alternativ dazu sind als bewegbare Erfassungseinrichtung bevorzugt zwei Spiegel vorgesehen, wobei die Scaneinrichtung feststehend angeordnet ist. Die zuletzt angegebene Möglichkeit ist vorzuziehen, da Spiegel einfacher zu bewegen sind als eine Scaneinrichtung.

Die Antriebseinrichtung ist bevorzugt ausserhalb des Mundstücks, d.h. im Griff der Scanvorrichtung, angeordnet. Durch diese Anordnung bleibt im Mundstück genügend Freiraum zur Bewegung der Erfassungseinrichtung.

Das Mundstück hat eine obere und eine untere Grundfläche, die durch eine umlaufende Seitenwand in Verbindung stehen. Die Seitenwand ist mit einer Öffnung in Richtung zu dem Griff vorgesehen, über welche Öffnung das Mundstück zu dem Griff übergeht. Griff und Mundstück bilden als Scanvorrichtung eine Einheit. Die in ihnen vorgesehenen technischen Einrichtungen sind getrennt vorgesehen, so dass eine Bewegung der Erfassungseinrichtung nicht gestört wird. So sind die Antriebseinrichtung und die Steuereinrichtung nicht im Mundstück vorgesehen. Die Steuereinrichtung ist bevorzugt auch ausserhalb vom Griff, aber natürlich mit den technischen Einrichtungen der Scanvorrichtung in Verbindung stehend, vorgesehen.

Das Mundstück hat weiterhin in seiner Draufsicht die Form eines gleichschenkligen Trapezes mit einer Grundlinie, die eine Breite gleich der Breite des Griffs der Scanvorrichtung hat, und mit einer weiteren Grundlinie, die eine Breite von etwa 55 bis 85 mm hat, die grösser als die Breite der einen Grundlinie ist, wobei die eine Grundlinie von der weiteren Grundlinie einen Abstand von etwa 45 bis 55 mm hat. Das Mundstück hat zudem in seiner Seitenansicht eine Höhe von etwa 20 mm, so dass es zwischen den Zähnen in den Mund eines Patienten einführbar ist.

Bevorzugt stehen Umhüllungen in verschiedenen Grössen zur Verfügung oder ist die Umhüllung derart ausgebildet, dass sie verstellt oder aufgefaltet werden kann. Somit können vorteilhaft verschieden grosse Gebisse gescannt werden. Somit ist die Umhüllung auf vorteilhafte Weise entfernbar und kann gereinigt und/oder sterilisiert werden.

Bevorzugt ist das Mundstück auch beheizbar, wobei die Heizung durch einen warmen Luftstrom im Innern des Mundstücks realisiert ist. Somit kann Beschlag im Mundstück verhindert werden.

Besonders bevorzugt sind im Mundstück zwei Spiegel vorgesehen. Dabei ist ein zentraler Spiegel an einem Ende eines ausserhalb des Griffs gelagerten Haltearms in Richtung zu der längeren Grundlinie des Trapezes und in Richtung einer Verlängerung des Griffs der Scanvorrichtung angebracht und ist ein äusserer Spiegel an einem Ende eines weiteren ausserhalb des Griffs gelagerten Haltearms in Richtung zu der längeren Grundlinie des Trapezes und an einem Schenkel des Trapezes entlanglaufend angebracht.

Der Haltearm für den zentralen Spiegel ist starr und für eine Translationsbewegung geeignet gelagert und der zentrale Spiegel am Ende des Haltearms ist in einem Halterahmen gelagert, wobei der zentrale Spiegel in dem Halterahmen um eine Achse parallel zu den Grundflächen des Mundstücks drehbar gelagert ist und der Halterahmen selbst am Ende des Haltearms um eine Achse senkrecht zu den Grundflächen des Mundstücks drehbar gelagert ist. Der weitere Haltearm für den äusseren Spiegel ist für eine Translationsbewegung und an seinem Lagerungsende für eine Rotationsbewegung um eine Achse senkrecht zu den Grundflächen des Mundstücks geeignet gelagert und weist mittig einen Drehpunkt auf, an welchem sie um eine Achse senkrecht zu den Grundflächen des Mundstücks gedreht werden kann. Weiterhin ist der äussere Spiegel am Ende des weiteren Haltearms um eine Achse parallel zu den Grundflächen des Mundstücks und um eine Achse senkrecht zu den Grundflächen des Mundstücks drehbar gelagert. Die Antriebseinrichtung bewirkt jeweils die Translationsbewegungen und Dreh- bzw. Rotationsbewegungen.

Der zentrale Spiegel hat bevorzugt drei Freiheitsgrade. Der äussere Spiegel hat bevorzugt wenigstens vier Freiheitsgrade.

Mit der feststehenden Scaneinrichtung und zwei durch die Antriebseinrichtung bewegbaren Spiegeln ist somit jede Stelle eines Zahnbogens einsehbar, sofern die beiden Spiegel genügend Bewegungsfreiheit haben, die durch die beschriebene Lagerung der Spiegel gegeben ist. Die Spiegel müssen nicht präzise angetrieben werden. Eine Scangenauigkeit hängt nicht von der genauen Kenntnis der Spiegelposition ab. Nur ein jeweiliger Einzelscan muss genau sein.

Die Erfindung betrifft auch ein Verfahren zum Scannen eines gesamten Kiefers mit einer erfindungsgemässen Scanvorrichtung, welches Verfahren vorzugsweise die folgenden Schritte aufweist:
- Einführen des Mundstücks der Scanvorrichtung in den Mund eines Patienten,
- Kalibrieren der Scanvorrichtung,
- Scannen und dreidimensionales Vermessen eines Ausschnitts des Zahnbogens im Mund,
- gleichzeitiges Scannen einer der Scanposition entsprechenden Kalibrierungsmarke auf der Umhüllung des Mundstücks bzw. dem Mundstück,
- automatisches Bewegen der Spiegel im Mundstück zu einer weiteren Scanposition und weiteres Scannen eines Ausschnitts des Zahnbogens im Mund einer der Scanposition entsprechenden Kalibrierungsmarke auf der Umhüllung des Mundstücks bzw. dem Mundstück,
- automatisches Wiederholen des vorangehenden Schritts, bis alle Ausschnitte des Zahnbogens im Mund gescannt sind und
- Zusammenfügen der Einzelscans zum Erhalten der vollständigen Kieferdaten.

Erfindungsgemäss erfolgt auf vorteilhafte Weise ein automatisches Weiterschalten von einer Scanposition zu einer anderen, ohne dass eine Bedienperson der Scanvorrichtung tätig werden muss. Es erfolgt ein relativ schnelles Scannen des gesamten Kiefers, ohne dass die Scanvorrichtung im Mund eines Patienten bewegt werden muss, was für einen Patienten eine grosse Erleichterung gegenüber einer durch eine Bedienperson manuell bewegte Scanvorrichtung im Mund ist.

Vorzugsweise weist das Verfahren auch den Schritt eines A-nalysierens der erhaltenen Kieferdaten zur Feststellung einer Vollständigkeit der Kieferdaten und, im Fall unvollständiger Kieferdaten und somit von Leerstellen bei den Kieferdaten, die Schritte eines jeweiligen automatischen Bewegens der Spiegel im Mundstück zu den den Leerstellen entsprechenden Scanpositionen und eines weiteren Scannens eines Ausschnitts des Zahnbogens im Mund einer der Scanposition entsprechenden Kalibrierungsmarke auf der Umhüllung des Mundstücks bzw. dem Mundstück und eines Zusammenfügens der zur Korrektur durchgeführten Einzelscans mit den bereits vorhanden Einzelscans.

Während das Zusammenfügen der Einzelscans im Fall des freihändigen Scannens zu Genauigkeitsproblemen führen kann, da kleine Fehler der Einzelscans aufaddiert werden, kann dieses Problem durch die Scanvorrichtung und das mit der Scanvorrichtung durchgeführte Verfahren gemäss der vorliegenden Erfindung auf einfache Weise behoben werden.

Das Kalibrieren der Scanvorrichtung weist die folgenden Schritte auf:
- ein- oder mehrmaliges Scannen der Innenseite des Kiefers bei gleichzeitigem Scannen jeweiliger Kalibrierungsmarken, während nur mittels des zentralen Spiegels die Scanposition an die Innenseite des Kiefers abgelenkt wird,
- Rotieren des zentralen Spiegels und somit Scannen des gesamten Zahnbogens zusammen mit jeweiligen Kalibrierungsmarken von innen,
- Zusammenfügen der Einzelscans zum Gesamtscan der Innenseite des Kiefers, wobei jeweils die Konturen der Zähne zusammenfügt werden,
- Analysieren der Lagen der Kalibrierungsmarken und Vergleichen mit Sollpositionen der Lagen zum Bestimmen einer Abweichung,
- Berechnen einer Entzerrungsvorschrift aus der Abweichung, wie die gemessenen Kalibrierungsmarken entzerrt werden müssen, damit sie mit den tatsächlichen Kalibrierungsmarken zur Deckung gebracht werden können, und
- Anwenden dieser Entzerrungsvorschrift auf den gemessenen Zahnbogen zur Korrektur der erhaltenen Kieferdaten.

Nach dem Kalibrieren werden die folgenden Daten für die Scans von Oberseite und Aussenseite des Zahnbogens an den gemessen Zahnbogen angefügt.

Da das Scannen der Innenseite des Zahnbogens sehr schnell geht, kann es auch mehrmals durchgeführt werden. Damit kann erkannt werden, ob die Kalibrierungsmarken während des Scannens gegenüber dem Zahnbogen verschoben wurden.

Eine Innenseite von Zähnen wird vorteilhafterweise unter Verwendung von nur einem Spiegel gescannt, der sich zentral in der Mitte des Mundraums befindet und so verkippt wird, dass er den Scan-Ausschnitt auf die Innenseite der Zähne lenkt.

Beim Scannen von Kauflächen wird der Scanbereich vorteilhafterweise von einem zentralen Spiegel nach aussen auf den zweiten Spiegel gelenkt, der senkrecht über bzw. unter den Zähnen liegt, und dann von dem zweiten Spiegel um ca. 90° auf die Kauffläche gelenkt.

Vorteilhafterweise wird der zweite Spiegel beim Scannen der Aussenseite von Zähnen weiter nach aussen als beim Scannen von Kauflächen positioniert und dann wird der Scanbereich von aussen an die Zahnflächen gelenkt.

Bei einem Mundstück, das oben und unten transparent ist, erfolgt vorteilhafterweise durch Verkippung der Spiegel ein Wechseln eines Scannens von Unter- und Oberkiefer.

Erfindungsgemäss ergibt sich somit der Vorteil eines schnellen, unkomplizierten und präzisen Scannens eines gesamten Kiefers.

Die angegebenen und weitere Merkmale und Einzelheiten der Erfindung werden einem Fachmann auf dem Gebiet aus der folgenden detaillierten Beschreibung und den beigefügten Zeichnungen klarer, die Merkmale der vorliegenden Erfindung anhand eines Beispiels darstellen und wobei
- Figur 1: eine Schrägansicht einer Scanvorrichtung gemäss der vorliegenden Erfindung darstellt,
- Figur 2: eine Schrägansicht eines Mundstücks mit Umhüllung und Kalibrierungsmarken der Scanvorrichtung gemäss der vorliegenden Erfindung darstellt,
- Figur 3: eine Draufsicht auf ein Mundstück der Scanvorrichtung mit Messköpfen gemäss der vorliegenden Erfindung darstellt,
- Figur 4: eine Seitenansicht eines Mundstücks der Scanvorrichtung mit Messköpfen gemäss der vorliegenden Erfindung darstellt,
- Figur 5: eine Draufsicht auf das Innere des Mundstücks der Scanvorrichtung gemäss der vorliegenden Erfindung darstellt,
- Figur 6: eine weitere Draufsicht auf das Innere des Mundstücks der Scanvorrichtung gemäss der vorliegenden Erfindung darstellt,
- Figur 7: eine Schrägansicht des offenen Mundstücks der Scanvorrichtung gemäss der vorliegenden Erfindung darstellt,
- Figur 8: eine weitere Schrägansicht des offenen Mundstücks der Scanvorrichtung gemäss der vorliegenden Erfindung darstellt,
- Figur 9: eine Schrägansicht eines zentralen Spiegels und seiner Halterung gemäss der vorliegenden Erfindung darstellt, und
- Figur 10: eine Schrägansicht eines äusseren Spiegels und seiner Halterung gemäss der vorliegenden Erfindung darstellt.

Im Folgenden wird die vorliegende Erfindung anhand bevorzugter Ausführungsformen unter Bezugnahme auf die Figuren detailliert erklärt werden.

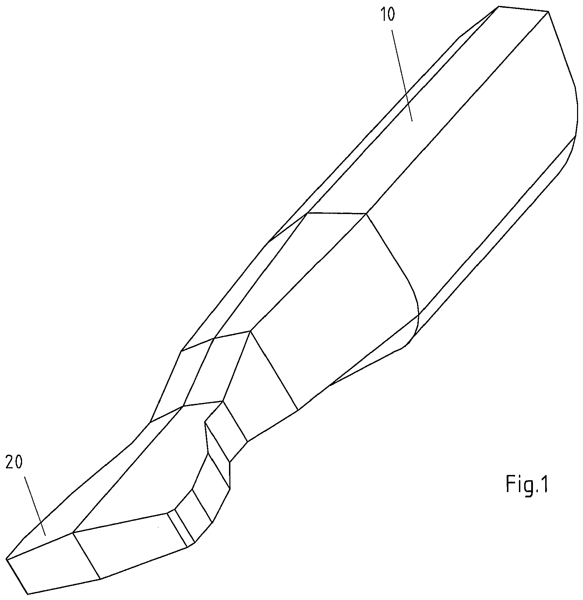

Figur 1 zeigt eine Schrägansicht eines allgemeinen Aufbaus einer erfindungsgemässen Scanvorrichtung, die von einer Bedienperson zu haltenden Griff 10 und ein mit diesem Griff 10 verbundenes Mundstück 20 zeigt. Das relativ grosse, zumindest teilweise transparente Mundstück 20 der Scanvorrichtung wird in den Mund eines Patienten eingeführt. Dort wird bei einer Aufnahme durch eine innerhalb des Griffs 10 angeordnete Scaneinrichtung jeweils ein Ausschnitt eines Zahnbogens im Mund gescannt und dreidimensional vermessen. Die Scaneinrichtung ist vorzugsweise eine Kamera oder ein 3D-Scanner, der über einen grossen Tiefenbereich, wie beispielsweise 100 mm, scannen kann. Sie kann nach dem Prinzip der Konfokalmikroskopie arbeiten.

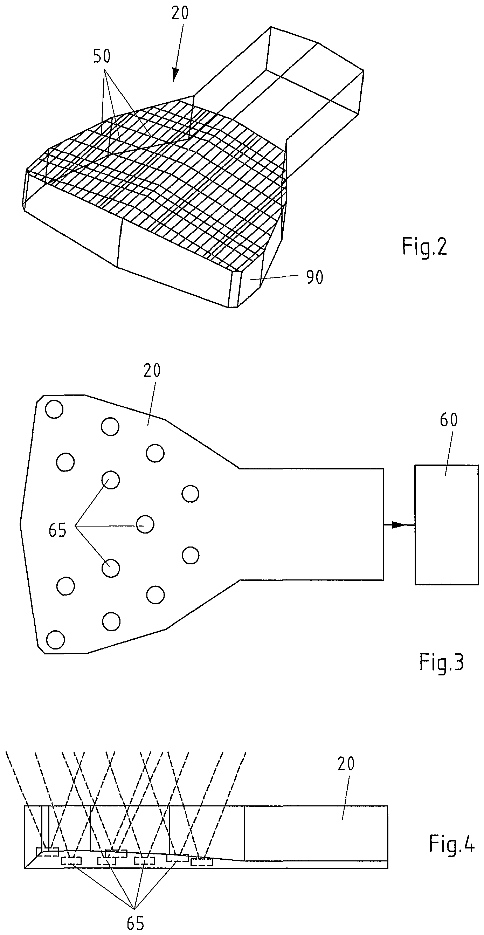

Wie es in der Figur 2 gezeigt ist, umgibt das transparente Mundstück 20 eine Umhüllung 90, auf der präzise Kalibrierungsmarken 50 aufgebracht sind, die zusammen mit einem jeweiligen Einzelscan gescannt werden. Die Kalibrierungsmarken 50 sind hier als kariertes Muster gezeigt, können aber auch ein anderes Muster aufweisen.

Es werden viele Einzelscans von verschiedenen Ausschnitten des Zahnbogens aufgenommen und zu einem Gesamtbild zusammengefügt. Erfindungsgemäss sind im Mündstück 20 technische Einrichtungen vorgesehen, die automatisch gesteuert gleichzeitig oder kurz nacheinander einen jeweiligen Einzelscan der verschiedenen Ausschnitte des Zahnbogens durchführen.

In den Figuren 3 und 4 ist ein Mundstück 20 einer Scanvorrichtung jeweils in einer Draufsicht und einer Seitenansicht gezeigt. Es ist zu sehen, dass im trapezförmigen Mundstück 20 eine Vielzahl von Messköpfen 65 so angeordnet ist, dass sie versetzt in zwei Reihen entlang den Schenkeln des Trapezes vorgesehen sind. Durch diese Anordnung überlappen sich Messbereiche, wie es insbesondere in der Figur 4 zu sehen ist. In Figur 3 ist zusätzlich angedeutet, wie von den Messköpfen 65 erfasste Daten zu einer Scaneinrichtung 60 weitergeleitet werden.

Gemäss den Figuren 3 und 4 ist die Vielzahl von Messköpfen 65 für die Scaneinrichtung 60 also derart positioniert, dass mit ihnen der gesamte Oberkiefer eines Patienten gleichzeitig gescannt wird. Zum Scannen des gesamten Unterkiefers muss die Scanvorrichtung umgekehrt werden. Es ist auch vorstellbar, dass Messköpfe 65 derart vorgesehen sind, dass sowohl Oberkiefer als auch Unterkiefer eines Patienten gleichzeitig gescannt werden können.

Ein ähnliches Prinzip ist beispielsweise für die Vermessung von Autos bekannt. Für den Einsatz zum Vermessen eines gesamten Oberkiefers und/oder Unterkiefers muss dieses Prinzip aber derart abgeändert werden, dass die Messköpfe als Miniaturoptiken, wie beispielsweise Endoskopoptiken, ausgebildet sind. Der Einsatz der Vielzahl von Messköpfen ermöglicht ein sehr schnelles gleichzeitiges Scannen eines gesamten Kiefers eines Patienten.

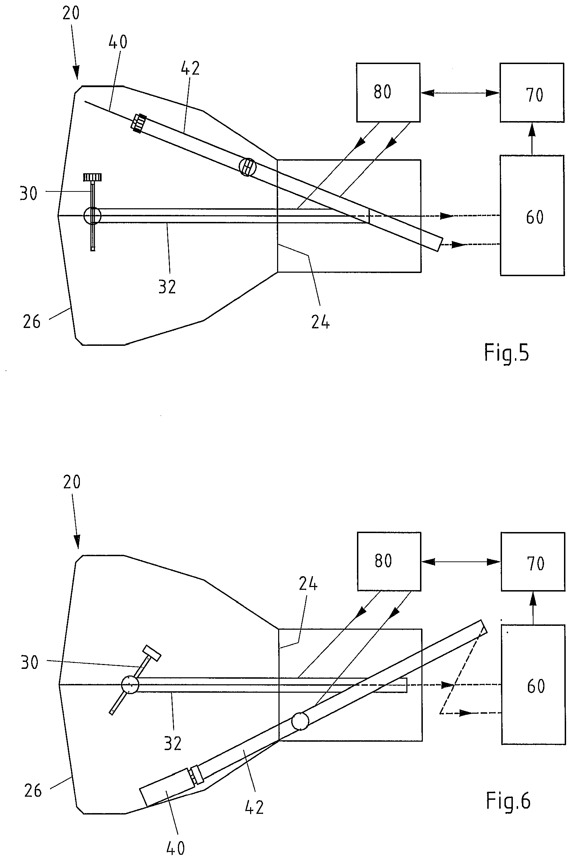

Alternativ oder zusätzlich sind erfindungsgemäss, wie es in der Figur 5 gezeigt ist, eine Antriebseinrichtung 80 und eine Steuereinrichtung 70 zusätzlich zu einer Scaneinrichtung 60 vorgesehen. Die Antriebseinrichtung 80 treibt eine Erfassungseinrichtung derart an, dass sie sich von einem Ausschnitt zu einem nächsten Ausschnitt des Zahnbogens bewegt, um einen jeweiligen Einzelscan durchzuführen, und die Steuereinrichtung 70 steuert die Antriebseinrichtung 80 derart, dass sie die Erfassungseinrichtung zu einer gewünschten Stelle des Zahnbogens bewegt.

Wie es beispielsweise in den Figuren 5 und 6 auch gezeigt ist, weist die Erfassungseinrichtung zwei Spiegel 30, 40 auf, die motorisch angetrieben so bewegt werden, dass mit ihnen ein Scanbereich an eine gewünschte Stelle des Zahnbogens abgelenkt wird.

In den Figuren 5 und 6 ist deutlich zu erkennen, dass das Mundstück 20 eine obere Grundfläche und eine untere Grundfläche hat, die durch eine umlaufende Seitenwand in Verbindung stehen. Die Seitenwand ist mit einer Öffnung 22 in Richtung zu dem von einer Bedienperson zu haltenden Griff 10 der Scanvorrichtung versehen, über welche Öffnung 22 das Mundstück 20 zu dem Griff 10 übergeht.

Das Mundstück 20 hat in seiner Draufsicht die Form eines gleichschenkligen Trapezes mit einer Grundlinie 24, die eine Breite gleich der Breite des Griffs 10 der Scanvorrichtung hat, und mit einer weiteren Grundlinie 26, die eine Breite von etwa 55 bis 85 mm hat, die grösser als die Breite der einen Grundlinie 24 ist, wobei die eine Grundlinie 24 von der weiteren Grundlinie 26 einen Abstand von etwa 45 bis 55 mm hat und wobei das Mundstück 20 in seiner Seitenansicht eine Höhe 28 von etwa 20 mm hat, so dass es zwischen den Zähnen in den Mund eines Patienten einführbar ist.

Wie es bereits angegeben ist, ist das Mundstück 20 mit einer Umhüllung 90 versehen. Es stehen Umhüllungen 90 in verschiedenen Grössen zur Verfügung. Die Umhüllung 90 kann auch derart ausgebildet sein, dass sie aufgefaltet werden kann, so dass sie zum Einführen in unterschiedlich grosse Mundräume von Patienten geeignet ist. Zusätzlich ist das Mundstück 20 beheizbar, wobei die Heizung durch einen warmen Luftstrom im Innern des Mundstücks 20 realisiert ist.

Aus den Figuren 5 bis 8 ist deutlich zu erkennen, dass im Mundstück 20 zwei Spiegel 30, 40 vorgesehen sind. Ein zentraler Spiegel 30 ist an einem Ende eines Haltearms 32 in Richtung zu der längeren Grundlinie 26 des Trapezes und in Richtung einer Verlängerung des Griffs 10 der Scanvorrichtung angebracht. Ein äusserer Spiegel 40 ist an einem Ende eines Haltearms 42 in Richtung zu der längeren Grundlinie 26 des Trapezes und an einem Schenkel des Trapezes entlanglaufend angebracht.

Der Haltearm 32 für den zentralen Spiegel 30 ist starr und für eine Translationsbewegung geeignet gelagert und der zentrale Spiegel 30 am Ende des Haltearms 32 ist in einem Halterahmen 34 gelagert, wobei der zentrale Spiegel 30 in dem Halterahmen 34 um eine Achse parallel zu den Grundflächen des Mundstücks 20 drehbar gelagert ist und der Halterahmen 34 selbst am Ende des Haltearms 32 um eine Achse senkrecht zu den Grundflächen des Mundstücks 20 drehbar gelagert ist. Der weitere Haltearm 42 für den äusseren Spiegel 40 ist für eine Translationsbewegung und an seinem Lagerungsende für eine Rotationsbewegung um eine Achse senkrecht zu den Grundflächen des Mundstücks 20 geeignet gelagert und weist mittig einen Drehpunkt auf, an welchem sie um eine Achse senkrecht zu den Grundflächen des Mundstücks 20 gedreht werden kann, und der äussere Spiegel 40 am Ende des weiteren Haltearms 42 ist um eine Achse parallel zu den Grundflächen des Mundstücks 20 und um eine Achse senkrecht zu den Grundflächen des Mundstücks 20 drehbar gelagert. Die Antriebseinrichtung 80 bewirkt die Translationsbewegungen und Dreh- bzw. Rotationsbewegungen.

Nachdem das Mundstück 20 gemäss einer bevorzugten Ausführungsform der vorliegenden Erfindung zusammen mit den Spiegeln 30, 40 und ihren Halterungen im Mundstück beschrieben worden ist, wird nun noch kurz auf die Figuren 5 bis 10 eingegangen.

Die Figuren 5 und 6 zeigen in einer Draufsicht jeweils ein Mundstück 20 mit Haltearmen 32 und 42, an denen jeweils ein zentraler Spiegel 30 und äusserer Spiegel 40 gelagert ist. Die beiden Figuren zeigen eine jeweils unterschiedliche Position der Haltearme 32, 42 und der Spiegel 30, 40.

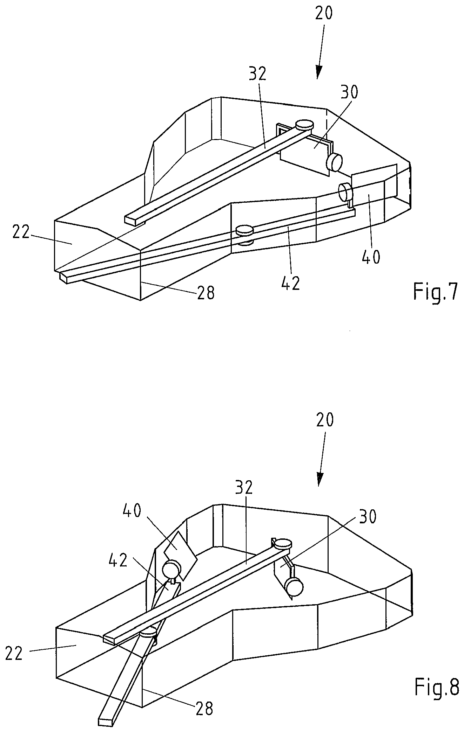

Die Figuren 7 und 8 zeigen in einer Schrägansicht jeweils ein Mundstück 20 mit Haltearmen 32 und 42, an denen jeweils ein zentraler Spiegel 30 und äusserer Spiegel 40 gelagert ist. Die beiden Figuren zeigen entsprechend den Figuren 3 und 4 eine jeweils unterschiedliche Position der Haltearme 32, 42 und der Spiegel 30, 40.

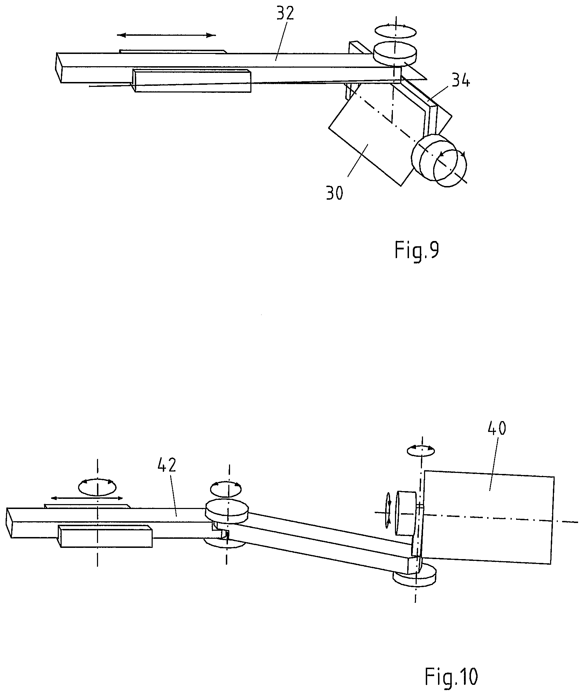

Schliesslich zeigen die Figuren 9 und 10 jeweils einen Haltearm 32 mit dem zentralen Spiegel 30 und einen Haltearm 42 mit dem äusseren Spiegel 40. In diesen Figuren ist die oben bereits angedeutete bewegbare und drehbare Lagerung der Spiegel 30, 40 gut zu erkennen.

Generell ist der Platz innerhalb des Mundstücks 20 knapp. Deshalb werden vorzugsweise die meisten Antriebselemente ausserhalb des Mundstücks 20 angeordnet. Antriebe bzw. Antriebselemente, die innerhalb des Mundstücks 20 liegen, müssen sehr kompakt ausgeführt sein.

Bei der vorliegenden Beschreibung sind der Spiegel 30 und der Spiegel 40 unterschiedlich aufgebaut. Der Spiegel 30 kann einfacher gestaltet werden als der Spiegel 40. Es kann aber auch vorteilhaft sein, beide Spiegel genau gleich zu gestalten - dann wären beide so gestaltet wie der beschriebene Spiegel 40. Dieser Spiegel kann auch alles, was der Spiegel 30 kann.

Insbesondere ist aus der Figur 9 zu erkennen, dass der Spiegel 30 drei Freiheitsgrade hat. Es gibt eine Translationsachse und zwei Rotationsachsen. Der Haltearm 32 ist in einer Linearführung geführt und kann motorisch verschoben werden. Die Verschiebung kann beispielsweise mit einem Spindelantrieb oder einem Linearmotor erfolgen. Lagerung und Antrieb dieses Haltearms 32 sind vorzugsweise ausserhalb des Mundstücks 20 angeordnet. Eine Rotationsachse betrifft den Halterahmen 34. Der an dem Haltearm 32 befestigte Halterahmen 34 ist drehbar. Er ist beispielsweise mit einem Miniaturkugellager gehalten. Der Antrieb kann beispielsweise, wie es in der Figur 9 skizziert ist, über eine Seite erfolgen. Eine Rückstellung kann mittels Rückstellfeder erreicht werden. Eine andere Möglichkeit wäre ein Miniaturmotor, wie beispielsweise ein Ultraschall-Antrieb. Ein solcher Miniaturmotor könnte in einem scheibenartig skizzierten Raum für den Antrieb untergebracht werden. Bei einer Lösung mit Seite kann der Antrieb dieser Achse ausserhalb des Mundstücks 20 angeordnet werden. Um eine weitere Rotationsachse muss der Spiegel 30 nur gekippt werden. Das wird vorteilhaft mit einem Miniaturmotor oder einem magnetischen Antrieb, wie es ein Drehspulinstrument aufweist, realisiert.

Weiterhin ist insbesondere aus der Figur 10 zu erkennen, dass der Spiegel 40 fünf Freiheitsgrade hat. Weniger Freiheitsgrade sind möglich, allerdings sind dann gewisse Partien im Mund eines Patienten schwer einsehbar. Nach heutigem Kenntnisstand sind minimal vier Freiheitsgrade nötig.

Es ist eine Translationsachse wie beim Spiegel 30 zu sehen. Weiterhin gibt es eine Rotationsachse bei der Translationsachse. Linearführung und -antrieb sind drehbar angeordnet. Antrieb und Lagerung können ausserhalb des Mundstücks angeordnet werden. Ein Antrieb erfolgt mit Miniaturmotor. Dann gibt es eine Rotationsachse gleich der weiteren Rotationsachse des Spiegels 30. Die weiteren Rotationsachsen haben Antriebe wie die erste Rotationsachse des Spiegels 30.

Es ist noch zu erwähnen, dass die Steuereinrichtung eine Berechnungs- und Auswerteeinrichtung zum Zusammenfügen der Einzelscans zum Erhalten der vollständigen Kieferdaten unter Berücksichtigung der gescannten Kalibrierungsmarken und somit der Position der Spiegel, zur Analyse der erhaltenen Kieferdaten zur Feststellung einer Vollständigkeit der Kieferdaten und zur Vervollständigung der Kieferdaten aufweist.

Nachdem vorangehend eine bevorzugte Ausführungsform einer Scanvorrichtung gemäss der vorliegenden Erfindung ausführlich beschrieben worden ist, wird nun nachfolgend ein mit dieser Scanvorrichtung durchgeführtes Scanverfahren beschrieben.

Zuerst wird das Mundstück 20 der Scanvorrichtung in den Mund eines Patienten eingeführt, um dann die Scanvorrichtung zu kalibrieren. Dann wird ein Ausschnitt des Zahnbogens im Mund gescannt und dreidimensional vermessen, während gleichzeitig eine der Scanposition entsprechende Kalibrierungsmarke 50 auf dem Mundstück 20 gescannt wird. Darauffolgend wird der Spiegel bzw. werden die Spiegel 30, 40 im Mundstück 20 automatisch zu einer weiteren Scanposition bewegt und wird ein weiterer Ausschnitt des Zahnbogens im Mund einer der Scanposition entsprechenden Kalibrierungsmarke 50 auf dem Mundstück 20 gescannt. Dieser Schritt wird automatisch wiederholt, bis alle Ausschnitte des Zahnbogens im Mund gescannt sind. Dann werden die Einzelscans zum Erhalten der vollständigen Kieferdaten zusammengefügt.

Um zwischen Unter- und Oberkiefer zu wechseln kann die Scanvorrichtung aus dem Mund herausgenommen, um 180° gedreht und umgekehrt wieder eingeführt werden. Durch entsprechende Verkippung der beiden Spiegel 30 und 40 kann aber ebenfalls zwischen Unter- und Oberkiefer gewechselt werden. Die Spiegel 30 und 40 können so das Licht nach unten statt nach oben ablenken. So können, ohne zwischenzeitlich die Scanvorrichtung aus dem Mund zu nehmen, sowohl Unter- als auch Oberkiefer gescannt werden. In diesem Fall muss das Mundstück oben und unten transparent sein und muss auf beiden Seiten Kalibrierungsmarken aufweisen.

Die Innenseite (lingual) kann vorteilhaft unter Verwendung von nur einem Spiegel gescannt werden. Dieser Spiegel 30 befindet sich dazu zentral in der Mitte des Mundraumes und wird so verkippt, dass er den Scan-Ausschnitt auf die Innenseite der Zähne lenkt.

Um die Kauflächen (okklusal) zu scannen wird der Scanbereich von einem zentralen Spiegel 30 erst nach aussen auf den zweiten Spiegel 40 gelenkt, der senkrecht über- bzw. unter den Zähnen liegt, und dann von diesem zweiten Spiegel 40 um ca. 90° auf die Kaufläche gelenkt.

Um die Aussenseite (bukkal) zu scannen, wird ähnlich wie bei der Kaufläche vorgegangen. Dabei wird aber der zweite Spiegel 40 weiter nach aussen positioniert und dann der Scanbereich von aussen an die Zahnfläche gelenkt.

Diese Aussenposition des zweiten Spiegels 40 ist dessen äusserste Position -sie bestimmt die minimale Grösse des Mundstücks.

Bei jedem Spiegel wird der Scanbereich natürlich gespiegelt - er wird also seitenverkehrt. Die beim Scannen erhaltenen Daten werden dadurch auch erst mal seitenverkehrt erfasst. Bei zwei Spiegeln 30 und 40 ist ein Scan wieder normal. Beim Auswerten muss man also wissen, wie viele Spiegel bei einem Einzelscan im Einsatz waren und muss dann ein entsprechendes Rückspiegeln durchführen.

Eine Reihenfolge eines Scannens ist prinzipiell frei wählbar. Gemäss einer bevorzugten Reihenfolge beginnt man mit einem Scannen der Innenseite, scannt dann die Kaufläche und schliesslich die Aussenseite.

Die erhaltenen Kieferdaten werden zur Feststellung einer Vollständigkeit der Kieferdaten analysiert. Im Fall unvollständiger Kieferdaten und somit von Leerstellen bei den Kieferdaten, wird der Spiegel bzw. werden die Spiegel 30, 40 im Mundstück 20 automatisch zu den den Leerstellen entsprechenden Scanpositionen bewegt. Es erfolgt ein weiteres Scannen eines Ausschnitts des Zahnbogens im Mund einer der Scanposition entsprechenden Kalibrierungsmarke 50 auf dem Mundstück 20 und schliesslich ein Zusammenfügen der zur Korrektur durchgeführten Einzelscans mit den bereits vorhandenen Einzelscans.

Das Kalibrieren erfolgt durch ein- oder mehrmaliges Scannen der Innenseite des Kiefers bei gleichzeitigem Scannen jeweiliger Kalibrierungsmarken 50, während nur mittels des zentralen Spiegels 30 die Scanposition an die Innenseite des Kiefers abgelenkt wird, ein Rotieren des zentralen Spiegels 30 und somit Scannen des gesamten Zahnbogens zusammen mit jeweiligen Kalibrierungsmarken 50 von innen, ein Zusammenfügen der Einzelscans zum Gesamtscan der Innenseite des Kiefers, wobei jeweils die Konturen der Zähne zusammenfügt werden, ein Analysieren der Lagen der Kalibrierungsmarken 50 und Vergleichen mit Sollpositionen der Lagen zum Bestimmen einer Abweichung, ein Berechnen einer Entzerrungsvorschrift aus der Abweichung, wie die gemessenen Kalibrierungsmarken 50 entzerrt werden müssen, damit sie mit den tatsächlichen Kalibrierungsmarken 50 zur Deckung gebracht werden können, und ein Anwenden dieser Entzerrungsvorschrift auf den gemessenen Zahnbogen zur Korrektur der erhaltenen Kieferdaten.

Nach dem Kalibrieren werden die folgenden erhaltenen Daten für die Scans von Oberseite und Aussenseite des Zahnbogens an den gemessen Zahnbogen angefügt werden.

Die vorliegende Erfindung zeigt eine Scanvorrichtung und ein Scanverfahren, wobei eine Bedienperson nur einmal die Scanvorrichtung im Mund eines Patienten plazieren muss, wonach das Scanverfahren automatisch abläuft.

Ein gesamter Ober- oder Unterkiefer kann vollständig vermessen werden, ohne das eine Scaneinrichtung relativ zum Kiefer bewegt werden muss. Das bedeutet, dass ein Bediener keine besondere Messstrategie verfolgen muss, um die Vermessung vollständig durchzuführen.

Vereinfacht ausgedrückt besteht das Konzept gemäss der vorliegenden Erfindung darin, dass eine ausserhalb der Mundhöhle angebrachte 3D-Vermessungskamera als Scaneinrichtung mit einer Messerweiterung für die Mundhöhle ist. Die 3D-Vermessungskamera würde mit einer anders gestalteten Messanordnung prinzipiell auch andere Geometrien vermessen können. Die Messerweiterung stellt somit eine "Verlängerung" dar, welche derart variabel gestaltet ist, dass viele Messperspektiven eingenommen werden können. Im Vergleich zu bekannten Systemen, wie beispielsweise der eingangs angegebenen "CEREC Omnicam" ersetzt das Spiegelsystem die manuelle Bewegung. Es ist auch denkbar, statt automatisch bewegbarer Spiegel eine Vielzahl von Messköpfen im Mundstück der Scanvorrichtung vorzusehen, die dort derart positioniert sind, dass ein gesamter Kiefer aufgenommen werden kann. Es ist auch möglich, bei einer Scanvorrichtung automatisch bewegbare Spiegel und mehrere Messköpfe vorzusehen.

## Patentansprüche

1. Scanvorrichtung mit einem von einer Bedienperson zu haltenden Griff (10) und einem Mundstück (20), das in den Mund eines Patienten einzuführen ist, wobei die Scanvorrichtung eine im Mundstück (20) vorgesehene Erfassungseinrichtung und eine Scaneinrichtung (60) aufweist, wobei die Erfassungseinrichtung in Verbindung mit der Scaneinrichtung (60) bei in den Mund eines Patienten eingeführtem Mundstück (20) bei einer Aufnahme jeweils einen Ausschnitt eines Zahnbogens im Mund scannt und dreidimensional vermisst, wobei viele Einzelscans von verschiedenen Ausschnitten des Zahnbogens aufgenommen und zu einem Gesamtbild zusammengefügt werden, wobei die Scanvorrichtung weiterhin folgendes aufweist:
eine Antriebseinrichtung (80), die die Erfassungseinrichtung derart antreibt, dass sie sich von einem Ausschnitt zu einem nächsten Ausschnitt des Zahnbogens bewegt, um einen jeweiligen Einzelscan durchzuführen, und
eine Steuereinrichtung (70), die die Antriebseinrichtung (80) derart steuert, dass sie die Erfassungseinrichtung zu einer gewünschten Stelle des Zahnbogens bewegt,
wobei die Erfassungseinrichtung wenigstens einen Spiegel (30, 40) aufweist, der im Mundstück (20) mittels der Antriebseinrichtung (80) motorisch angetrieben so bewegt wird, dass mit ihm ein Scanbereich an eine gewünschte Stelle des Zahnbogens abgelenkt wird, **dadurch gekennzeichnet, dass** für das Mundstück (20) eine Umhüllung (90) vorgesehen ist, die wenigstens teilweise transparent ist, wobei auf die Umhüllung (90) des Mundstücks (20) präzise Kalibrierungsmarken (50) aufgebracht sind, die zusammen mit einem jeweiligen Einzelscan gescannt werden.

2. Scanvorrichtung nach Anspruch 1, **dadurch gekennzeichnet, dass** der wenigstens eine Spiegel (30, 40) wenigstens drei Freiheitsgrade hat, wodurch mittels der Scaneinrichtung (60) im Mund eines Patienten lingual und/oder okklusal und/oder bukkal gescannt werden kann.

3. Scanvorrichtung nach Anspruch 1 oder 2, **dadurch gekennzeichnet, dass** die Steuereinrichtung (70) eine Berechnungs- und Auswerteeinrichtung zum Zusammenfügen der Einzelscans zum Erhalten vollständiger Kieferdaten unter Berücksichtigung der gescannten Kalibrierungsmarken (50) und somit der jeweiligen Position des Spiegels oder der Spiegel (30, 40), zur Analyse der erhaltenen Kieferdaten zur Feststellung einer Vollständigkeit der Kieferdaten und zur Vervollständigung der Kieferdaten aufweist.

4. Scanvorrichtung nach einem der vorangehenden Ansprüche, **dadurch gekennzeichnet, dass** die Scaneinrichtung (60) ein 3D-Scanner ist, der über einen grossen Tiefenbereich, wie beispielsweise 100 mm, scannen kann.

5. Scanvorrichtung nach einem der Ansprüche 1 bis 4, **dadurch gekennzeichnet, dass** ein Spiegel (30) vorgesehen ist und die Scaneinrichtung (60) bewegbar angeordnet ist.

6. Scanvorrichtung nach einem der Ansprüche 1 bis 4, **dadurch gekennzeichnet, dass** zwei Spiegel (30, 40) vorgesehen sind und die Scaneinrichtung (60) feststehend angeordnet ist.

7. Scanvorrichtung nach einem der vorangehenden Ansprüche, **dadurch gekennzeichnet, dass** die Antriebseinrichtung (80) ausserhalb des Mundstücks (20) angeordnet ist.

8. Scanvorrichtung nach einem der vorangehenden Ansprüche, **dadurch gekennzeichnet, dass** das Mundstück (20) eine obere und eine untere Grundfläche hat, die durch eine umlaufende Seitenwand in Verbindung stehen, wobei die Seitenwand mit einer Öffnung (22) in Richtung zu dem Griff (10) der Scanvorrichtung versehen ist, über welche Öffnung (22) das Mundstück (20) zu dem Griff (10) übergeht.

9. Scanvorrichtung nach einem der vorangehenden Ansprüche, **dadurch gekennzeichnet, dass** das Mundstück (20) in seiner Draufsicht die Form eines gleichschenkligen Trapezes mit einer Grundlinie (24) hat, die eine Breite gleich der Breite des Griffs (10) der Scanvorrichtung hat, und mit einer weiteren Grundlinie (26), die eine Breite von etwa 55 bis 85 mm hat, die grösser als die Breite der einen Grundlinie (24) ist, wobei die eine Grundlinie (24) von der weiteren Grundlinie (26) einen Abstand von etwa 45 bis 55 mm hat und wobei das Mundstück (20) in seiner Seitenansicht eine Höhe (28) von etwa 20 mm hat, so dass es zwischen den Zähnen in den Mund eines Patienten einführbar ist.

10. Scanvorrichtung nach einem der vorangehenden Ansprüche, **dadurch gekennzeichnet, dass** Umhüllungen (90) in verschiedenen Grössen zur Verfügung stehen oder die Umhüllung (90) derart ausgebildet, dass sie verstellbar oder auffaltbar ist.

11. Scanvorrichtung nach einem der vorangehenden Ansprüche, **dadurch gekennzeichnet, dass** das Mundstück (20) beheizbar ist, wobei die Heizung durch einen warmen Luftstrom im Innern des Mundstücks (20) realisiert ist.

12. Scanvorrichtung nach einem der Ansprüche 6 bis 11, **dadurch gekennzeichnet, dass** die zwei Spiegel (30, 40) einen an einem Ende eines ausserhalb des Griffs (10) gelagerten starren Haltearms (32) in Richtung zu der längeren Grundlinie (26) des Trapezes und in Richtung einer Verlängerung des Griffs (10) der Scanvorrichtung angebrachten zentralen Spiegel (30) und einen an einem Ende eines weiteren ausserhalb des Griffs (10) gelagerten Haltearms (42) in Richtung zu der längeren Grundlinie (26) des Trapezes und an einem Schenkel des Trapezes entlanglaufend angebrachten äusseren Spiegel (40) aufweisen.

13. Scanvorrichtung nach Anspruch 12, **dadurch gekennzeichnet, dass** der Haltearm (32) für den zentralen Spiegel (30) starr ist und für eine Translationsbewegung geeignet gelagert ist und der zentrale Spiegel (30) am Ende des Haltearms (32) in einem Halterahmen (34) gelagert ist, wobei der zentrale Spiegel (30) in dem Halterahmen (34) um eine Achse parallel zu den Grundflächen des Mundstücks (20) drehbar gelagert ist und der Halterahmen (34) selbst am Ende des Haltearms (32) um eine Achse senkrecht zu den Grundflächen des Mundstücks (20) drehbar gelagert ist, und der weitere Haltearm (42) für den äusseren Spiegel (40) für eine Translationsbewegung und an seinem Lagerungsende für eine Rotationsbewegung um eine Achse senkrecht zu den Grundflächen des Mundstücks (20) geeignet gelagert ist und mittig einen Drehpunkt aufweist, an welchem sie um eine Achse senkrecht zu den Grundflächen des Mundstücks (20) gedreht werden kann, und der äussere Spiegel (40) am Ende des weiteren Haltearm s (42) um eine Achse parallel zu den Grundflächen des Mundstücks (20) und um eine Achse senkrecht zu den Grundflächen des Mundstücks (20) drehbar gelagert ist, wobei die Antriebseinrichtung (80) die Translationsbewegungen und Dreh- bzw. Rotationsbewegungen bewirkt.

14. Scanvorrichtung nach Anspruch 12 oder 13, **dadurch gekennzeichnet, dass** der zentrale Spiegel (30) drei Freiheitsgrade hat.

15. Scanvorrichtung nach einem der Ansprüche 12 bis 14, **dadurch gekennzeichnet, dass** der äussere Spiegel (40) wenigstens vier Freiheitsgrade hat.

16. Verfahren zum Scannen eines gesamten Kiefers mit einer erfindungsgemässen Scanvorrichtung nach einem der vorangehenden Ansprüche, welches Verfahren die folgenden Schritte aufweist:
- Einführen des Mundstücks (20) der Scanvorrichtung in den Mund eines Patienten,
- Kalibrieren der Scanvorrichtung,
- Scannen und dreidimensionales Vermessen eines Ausschnitts des Zahnbogens im Mund mittels der Scanvorrichtung,
- gleichzeitiges Scannen einer der Scanposition entsprechenden Kalibrierungsmarke (50) auf der Umhüllung (90) des Mundstücks (20),
- automatisches Bewegen des Spiegels oder der Spiegel (30, 40) im Mundstück (20) zu einer weiteren Scanposition und weiteres Scannen eines Ausschnitts des Zahnbogens im Mund einer der Scanposition entsprechenden Kalibrierungsmarke (50) auf der Umhüllung (90) des Mundstücks (20),
- automatisches Wiederholen des vorangehenden Schritts, bis alle Ausschnitte des Zahnbogens im Mund gescannt sind und
- Zusammenfügen der Einzelscans zum Erhalten der vollständigen Kieferdaten.

17. Verfahren nach Anspruch 16, **dadurch gekennzeichnet, dass** es weiterhin folgende Schritte aufweist:
- Analysieren der erhaltenen Kieferdaten zur Feststellung einer Vollständigkeit der Kieferdaten und,
im Fall unvollständiger Kieferdaten und somit von Leerstellen bei den Kieferdaten,
- jeweiliges automatisches Bewegen des Spiegels oder der Spiegel (30, 40) im Mundstück (20) zu den den Leerstellen entsprechenden Scanpositionen,
- weiteres Scannen eines Ausschnitts des Zahnbogens im Mund einer der Scanposition entsprechenden Kalibrierungsmarke (50) auf der Umhüllung (90) des Mundstücks (20), und
- Zusammenfügen der zur Korrektur durchgeführten Einzelscans mit den bereits vorhandenen Einzelscans.

18. Verfahren nach Anspruch 16 oder 17, **dadurch gekennzeichnet, dass** das Kalibrieren folgende Schritte aufweist:
- ein- oder mehrmaliges Scannen der Innenseite des Kiefers bei gleichzeitigem Scannen jeweiliger Kalibrierungsmarken (50), während nur mittels des zentralen Spiegels (30) die Scanposition an die Innenseite des Kiefers abgelenkt wird,
- Rotieren des zentralen Spiegels (30) und somit Scannen des gesamten Zahnbogens zusammen mit jeweiligen Kalibrierungsmarken (50) von innen,
- Zusammenfügen der Einzelscans zum Gesamtscan der Innenseite des Kiefers, wobei jeweils die Konturen der Zähne zusammenfügt werden,
- Analysieren der Lagen der Kalibrierungsmarken (50) und Vergleichen mit Sollpositionen der Lagen zum Bestimmen einer Abweichung,
- Berechnen einer Entzerrungsvorschrift aus der Abweichung, wie die gemessenen Kalibrierungsmarken (50) entzerrt werden müssen, damit sie mit den tatsächlichen Kalibrierungsmarken (50) zur Deckung gebracht werden können, und
- Anwenden dieser Entzerrungsvorschrift auf den gemessenen Zahnbogen zur Korrektur der erhaltenen Kieferdaten.

19. Verfahren nach Anspruch 18, **dadurch gekennzeichnet, dass** nach dem Kalibrieren die folgenden Daten für die Scans von Oberseite und Aussenseite des Zahnbogens an den gemessen Zahnbogen angefügt werden.

20. Verfahren nach einem der Ansprüche 16 bis 19, **dadurch gekennzeichnet, dass** eine Innenseite von Zähnen unter Verwendung von nur einem Spiegel (30) gescannt wird, der sich zentral in der Mitte des Mundraums befindet und so verkippt wird, dass er den Scan-Ausschnitt auf die Innenseite der Zähne lenkt.

21. Verfahren nach einem der Ansprüche 16 bis 20, **dadurch gekennzeichnet, dass** beim Scannen von Kauflächen der Scanbereich von einem zentralen Spiegel (30) nach aussen auf den zweiten Spiegel (40) gelenkt wird, der senkrecht über bzw. unter den Zähnen liegt, und dann von dem zweiten Spiegel (40) um ca. 90° auf die Kauffläche gelenkt wird.

22. Verfahren nach Anspruch 21, **dadurch gekennzeichnet, dass** der zweite Spiegel (40) beim Scannen der Aussenseite von Zähnen weiter nach aussen als beim Scannen von Kauflächen positioniert wird und dann der Scanbereich von aussen an die Zahnflächen gelenkt wird.

23. Verfahren nach einem der Ansprüche 16 bis 22, **dadurch gekennzeichnet, dass** bei einem Mundstück (20), das oben und unten transparent ist, durch Verkippung der Spiegel (30, 40) ein Wechseln eines Scannens von Unter- und 0-berkiefer erfolgt.

## Claims

1. Scanning device with a handle (10) to be held by an operator and a mouthpiece (20) to be inserted into the mouth of a patient, wherein the scanning device comprises a detection apparatus and a scanning apparatus (60) provided in the mouthpiece (20), wherein, when the mouthpiece (20) is inserted into the mouth of a patient, during each recording the detection apparatus in conjunction with the scanning apparatus (60) scans and three-dimensionally measures one section of a dental arch in the mouth, wherein many individual scans of different sections of the dental arch are recorded and combined into one overall image of the dental arch, wherein the scanning device further comprises the following:
a drive apparatus (80), which drives the detection apparatus in such a way that it moves from one section to a next section of the dental arch to perform a respective single scan, and
a control apparatus (70), which controls the drive apparatus (80) in such a way that it moves the detection apparatus to a desired location of the dental arch,
wherein the detection apparatus comprises at least one mirror (30, 40), which is moved in a motor-driven manner in the mouthpiece (20) by means of the drive apparatus (80) in such a way that said mirror deflects a scan area onto a desired location of the dental arch, **characterized in that** a sheath (90), which is at least partially transparent, is provided for the mouthpiece (20), wherein precise calibration marks (50) are applied to the sheath (90) of the mouthpiece (20) that are scanned together with a respective individual scan.

2. Scanning device according to Claim 1, **characterized in that** the at least one mirror (30, 40) has at least three degrees of freedom, whereby lingual and/or occlusal and/or buccal scans can be performed in the mouth of a patient by means of the scanning apparatus (60) .

3. Scanning device according to Claim 1 or 2, **characterized in that** the control apparatus (70) comprises a calculation and evaluation device for assembling the individual scans to obtain complete information about the jaw while taking into account the scanned calibration marks (50) and thus the respective position of the mirror or the mirrors (30, 40), for the purpose of analyzing the obtained jaw data, to determine the completeness of the jaw data and to complete the jaw data.

4. Scanning device according to any of the preceding claims, **characterized in that** the scanning apparatus (60) is a 3D scanner that has the ability to scan over a large depth range, such as 100 mm for example.

5. Scanning device according to any of Claims 1 to 4, **characterized in that** a mirror (30) is provided and the scanning apparatus (60) is arranged in a movable manner.

6. Scanning device according to any of Claims 1 to 4, **characterized in that** two mirrors (30, 40) are provided and the scanning apparatus (60) is arranged in a fixed manner.

7. Scanning device according to any of the preceding claims, **characterized in that** the drive apparatus (80) is disposed outside the mouthpiece (20).

8. Scanning device according to any of the preceding claims, 5 **characterized in that** the mouthpiece (20) has an upper and a lower base surface, which are connected by a circumferential side wall, wherein the side wall is provided with an opening (22) in the direction toward the handle (10) of the scanning device, via which opening (22) the mouthpiece (20) transitions into the handle (10).

9. Scanning device according to any of the preceding claims, **characterized in that**, in plan view, the mouthpiece (20) is the shape of an isosceles trapezoid with one base line (24), the width of which is equal to the width of the handle (10) of the scanning apparatus, and with another base line (26), the width of which is about 55 to 85 mm, which is greater than the width of the one base line (24), wherein the one baseline (24) is at a distance of about 45 to 55 mm from the other base line (26) and wherein, in side view, the mouthpiece (20) has a height (28) of about 20 mm, allowing it to be inserted between the teeth into the mouth of a patient.

10. Scanning device according to any of the preceding claims, **characterized in that** sheaths (90) are available in a variety of sizes or the sheath (90) is configured in such a way that it is adjustable or unfolded.

11. Scanning device according to any of the preceding claims, **characterized in that** the mouthpiece (20) is heatable, wherein heating is realized by a stream of warm air in the interior of the mouthpiece (20).

12. Scanning device according to any of Claims 6 to 11, **characterized in that** the two mirrors (30, 40) comprise a central mirror (30) attached on one end of a rigid support arm (32), which is mounted outside the handle (10) in the direction toward the longer base line (26) of the trapezoid and in the direction of an extension of the handle (10) of the scanning device, and an external mirror (40) attached on one end of another support arm (42), which is mounted outside the handle (10) in the direction toward the longer base line (26) of the trapezoid and extending along one leg of the trapezoid.

13. Scanning device according to Claim 12, **characterized in that** the support arm (32) for the central mirror (30) is rigid and suitably mounted for a translational movement and the central mirror (30) is mounted on the end of the support arm (32) in a supporting frame (34), wherein the central mirror (30) is mounted in the supporting frame (34) in such a way that is rotatable about an axis parallel to the base surfaces of the mouthpiece (20) and the supporting frame (34) itself is mounted on the end of the support arm (32) in such a way that is rotatable about an axis perpendicular to the base surfaces of the mouthpiece (20), and the other support arm (42) for the external mirror (40) is suitably mounted for a translational movement and, at its mounting end, for a rotational movement about an axis perpendicular to the base surfaces of the mouthpiece (20) and centrally comprises a pivot point, on which it can be rotated about an axis perpendicular to the base surfaces of the mouthpiece (20), and the external mirror (40) is mounted on the end of the other support arm (42) in such a way that is rotatable about an axis parallel to the base surfaces of the mouthpiece (20) and about an axis perpendicular to the base surfaces of the mouthpiece (20), wherein the drive apparatus (80) produces the translational movements and the rotational movements.

14. Scanning device according to Claim 12 or 13, **characterized in that** the central mirror (30) has three degrees of freedom.

15. Scanning device according to any of Claims 12 to 14, **characterized in that** the external mirror (40) has at least four degrees of freedom.

16. Method for scanning an entire jaw with a scanning device according to any of the preceding claims, whereby the method comprises the following steps:
- insertion of the mouthpiece (20) of the scanning device into the mouth of a patient,
- calibration the scanning device,
- scanning and three-dimensional measurement of a section of the dental arch in the mouth by means of the scanning device,
- simultaneous scanning of a calibration mark (50) on the sheath (90) of the mouthpiece (20), whereby the calibration mark corresponds to the scan position,
- automatic movement of the mirror or mirrors (30, 40) in the mouthpiece (20) to another scan position and further scanning of a section of the dental arch in the mouth in the region of a calibration mark (50) on the sheath (90) of the mouthpiece (20) corresponding to the scan position,
- automatic repetition of the preceding step until all sections of the dental arch in the mouth are scanned, and
- combination of the individual scans to obtain the complete jaw data.

17. Method according to Claim 16, **characterized in that** it further comprises the following steps:
- analysis of the obtained jaw data to determine the completeness of the jaw data and, in the event that the jaw data is incomplete, determination of gaps in the jaw data,
- appropriate automatic movement of the mirror or mirrors (30, 40) in the mouthpiece (20) to the scan positions corresponding to the gaps,
- further scanning of a section of the dental arch in the mouth in the region of a calibration mark (50) on the sheath (90) of the mouthpiece (20) corresponding to the scan position, and
- combination of the individual scans conducted for the purpose of correction with the existing individual scans.

18. Method according to Claim 16 or 17, **characterized in that** the calibration comprises the following steps:
- one-time or multiple scanning of the inner side of the jaw with simultaneous scanning of the corresponding calibration marks (50), whereby the scan position is deflected to the inner side of the jaw only by means of the central mirror (30),
- rotating the central mirror (30) and thereby scanning the entire dental arch from the inner side along with the corresponding calibration marks (50),
- combination of the individual scans to form the overall scan of the inner side of the jaw, wherein the contours of the teeth are respectively combined,
- analysis of the locations of the calibration marks (50) and comparison to target positions of the locations to detect a deviation,
- calculation of a distortion correction rule from the deviation, specifying how the measured calibration marks (50) have to be corrected so that they can be brought into agreement with the actual calibration marks (50), and
- application of this distortion correction rule to the measured dental arch to correct the obtained jaw data.

19. Method according to Claim 18, **characterized in that**, after calibration, the following data for the scans of the upper side and the outer side of the dental arch is added to the measured dental arch.

20. Method according to any of Claims 16 to 19, **characterized in that** an inner side of the teeth is scanned by using only one mirror (30), which is located centrally in the middle of the oral cavity and is tilted in such a way that it directs the scanning section onto the inner side of the teeth.

21. Method according to any of Claims 16 to 20, **characterized in that**, when scanning chewing surfaces, the scan area is directed outward by a central mirror (30) onto the second mirror (40) located vertically above or below the teeth, and then deflected approximately 90° by the second mirror (40) onto the chewing surface.

22. Method according to Claim 21, **characterized in that**, when scanning the outer side of the teeth, the second mirror (40) is positioned further to the outside than when scanning chewing surfaces and the scan area is then directed onto the tooth surfaces from the outside.

23. Method according to any of Claims 16 to 22, **characterized in that**, in the case of a mouthpiece (20) that is transparent at the top and at the bottom, scanning is switched from the lower to the upper jaw by tilting the mirrors (30, 40).

## Revendications

1. Dispositif de balayage, comprenant une poignée (10) destinée à être tenue par un opérateur et un embout buccal (20) destiné à être introduit dans la bouche d'un patient, le dispositif de balayage présentant un dispositif d'acquisition disposé dans l'embout buccal (20) et un dispositif de balayage (60) ; lorsque l'embout buccal (20) est introduit dans la bouche d'un patient, le dispositif d'acquisition associé au dispositif de balayage (60) balaye dans la bouche et mesure en trois dimensions un segment d'une arcade dentaire au cours d'une même prise de vue, de nombreux clichés individuels de différents segments de l'arcade dentaire étant effectués et assemblés pour former une image globale, le dispositif de balayage présentant en outre les éléments suivants :
- un dispositif d'entraînement (80), qui entraîne le dispositif d'acquisition de sorte qu'il se déplace d'un segment de l'arcade dentaire à un autre, afin de réaliser un balayage individuel respectif, et
- un dispositif de commande (70) qui commande le dispositif d'entraînement (80) de sorte qu'il déplace le dispositif d'acquisition vers un emplacement souhaité de l'arcade dentaire ;
- le dispositif d'acquisition présentant au moins un miroir (30, 40) qui est déplacé de façon motorisée dans l'embout buccal (20) au moyen du dispositif d'entraînement (80) de sorte qu'une zone de balayage soit déviée par celui-ci vers un emplacement souhaité de l'arcade dentaire, **caractérisé en ce qu'**une enveloppe (90) est prévue pour l'embout buccal (20), laquelle est au moins partiellement transparente, des repères d'étalonnage (50) précis appliqués sur l'enveloppe (90) de l'embout buccal (20) étant balayés conjointement à un balayage individuel respectif.

2. Dispositif de balayage selon la revendication 1, **caractérisé en ce que** l'au moins un miroir (30, 40) possède au moins trois degrés de liberté, moyennant quoi le dispositif de balayage (60) peut effectuer, dans la bouche d'un patient, un balayage lingual et/ou occlusal et/ou buccal.

3. Dispositif de balayage selon la revendication 1 ou 2, **caractérisé en ce que** le dispositif de commande (70) présente un dispositif de calcul et d'évaluation pour l'assemblage des clichés individuels afin d'obtenir des données de mâchoire complètes compte tenu des repères d'étalonnage (50) balayés et ainsi de la position respective du ou des miroirs (30, 40), pour l'analyse des données de mâchoire obtenues afin de déterminer l'exhaustivité des données de mâchoire et de compléter les données de mâchoire.

4. Dispositif de balayage selon l'une des revendications précédentes, **caractérisé en ce que** le dispositif de balayage (60) est un scanner en 3D, qui peut effectuer un balayage sur une vaste plage de profondeur, comme par exemple 100 mm.

5. Dispositif de balayage selon l'une des revendications 1 à 4, **caractérisé en ce qu'**il est prévu un miroir (30) et **en ce que** le dispositif de balayage (60) est disposé mobile.

6. Dispositif de balayage selon l'une des revendications 1 à 4, **caractérisé en ce qu'**il est prévu deux miroirs (30, 40) et **en ce que** le dispositif de balayage (60) est disposé de manière fixe.

7. Dispositif de balayage selon l'une des revendications précédentes, **caractérisé en ce que** le dispositif d'entraînement (80) est disposé à l'extérieur de l'embout buccal (20).

8. Dispositif de balayage selon l'une des revendications précédentes, **caractérisé en ce que** l'embout buccal (20) possède des surfaces de base supérieure et inférieure qui sont reliées par une paroi latérale périphérique, la paroi latérale présentant une ouverture (22) dirigée vers la poignée (10) du dispositif de balayage, au niveau de laquelle ouverture (22) l'embout buccal (20) se transforme en poignée (10).

9. Dispositif de balayage selon l'une des revendications précédentes, **caractérisé en ce que** l'embout buccal (20) possède, vu de dessus, la forme d'un trapèze isocèle présentant une ligne de base (24) dont la largeur est identique à la largeur de la poignée (10) du dispositif de balayage, et une autre ligne de base (26) dont la largeur est d'environ 55 à 85 mm et est supérieure à la largeur de la ligne de base (24), la ligne de base (24) étant séparée de l'autre ligne de base (26) selon une distance d'environ 45 à 55 mm, et l'embout buccal (20) possédant, vu de côté, une hauteur (28) d'environ 20 mm, lui permettant d'être introduit entre les dents dans la bouche d'un patient.

10. Dispositif de balayage selon l'une des revendications précédentes, **caractérisé en ce qu'**il existe des enveloppes (90) de tailles différentes ou **en ce que** l'enveloppe (90) est formée de manière à être rétractable ou dépliable.

11. Dispositif de balayage selon l'une des revendications précédentes, **caractérisé en ce que** l'embout buccal (20) peut être chauffé, le chauffage étant réalisé par le biais d'un courant d'air chaud dans l'intérieur de l'embout buccal (20).

12. Dispositif de balayage selon l'une des revendications 6 à 11, **caractérisé en ce que** les deux miroirs (30, 40) présentent un miroir central (30) disposé au niveau d'une extrémité d'un bras de support (32) fixe placé à l'extérieur de la poignée (10) en direction de la ligne de base la plus longue (26) du trapèze et en direction d'un prolongement de la poignée (10) du dispositif de balayage, ainsi qu'un miroir extérieur (40) disposé au niveau d'une extrémité d'un autre bras de support (42) disposé à l'extérieur de la poignée (10) en direction de la ligne de base la plus longue (26) du trapèze et s'étendant le long d'une branche du trapèze.

13. Dispositif de balayage selon la revendication 12, **caractérisé en ce que** le bras de support (32) pour le miroir central (30) est fixe et est disposé de manière à pouvoir réaliser un mouvement de translation et le miroir central (30) est disposé au niveau de l'extrémité du bras de support (32) dans un cadre de support (34), le miroir central (30) étant disposé pour pouvoir tourner dans le cadre de support (34) autour d'un axe parallèle à la surface de base de l'embout buccal (20) et le cadre de support (34) lui-même étant disposé de manière à pouvoir tourner au niveau de l'extrémité du bras de support (32) autour d'un axe perpendiculaire à la surface de base de l'embout buccal (20), l'autre bras de support (42) pour le miroir extérieur (40) étant disposé pour pouvoir réaliser un mouvement de translation et, au niveau de son extrémité d'appui, un mouvement de rotation autour d'un axe perpendiculaire à la surface de base de l'embout buccal (20) et présentant au milieu un point de rotation au niveau duquel il peut tourner autour d'un axe perpendiculaire à la surface de base de l'embout buccal (20), et le miroir extérieur (40) étant disposé de manière à pouvoir tourner au niveau de l'extrémité de l'autre bras de support (42) autour d'un axe parallèle à la surface de base de l'embout buccal (20) et autour d'un axe perpendiculaire à la surface de base de l'embout buccal (20), le dispositif d'entraînement (80) provoquant les mouvements de translation et les mouvements de rotation.

14. Dispositif de balayage selon la revendication 12 ou 13, **caractérisé en ce que** le miroir central (30) possède trois degrés de liberté.

15. Dispositif de balayage selon l'une des revendications 12 à 14, **caractérisé en ce que** le miroir extérieur (40) possède au moins quatre degrés de liberté.

16. Procédé de balayage d'une mâchoire complète avec un dispositif de balayage selon l'invention selon l'une des revendications précédentes, lequel procédé présente les étapes suivantes :
- introduction de l'embout buccal (20) du dispositif de balayage dans la bouche d'un patient,
- étalonnage du dispositif de balayage,
- balayage et mesure en trois dimensions d'un segment de l'arcade dentaire dans la bouche au moyen du dispositif de balayage,
- balayage simultané d'un repère d'étalonnage (50) présent sur l'enveloppe (90) de l'embout buccal (20) et correspondant à la position de balayage,
- déplacement automatique du ou des miroirs (30, 40) à l'intérieur de l'embout buccal (20) vers une autre position de balayage, et balayage supplémentaire d'un segment de l'arcade dentaire dans la bouche d'un repère d'étalonnage (50) présent sur l'enveloppe (90) de l'embout buccal (20) et correspondant à la position de balayage,
- répétition automatique de l'étape précédente, jusqu'à ce que tous les segments de l'arcade dentaire de la bouche soient balayés, et
- assemblage des clichés individuels pour obtenir les données de mâchoire complètes.

17. Procédé selon la revendication 16, **caractérisé en ce qu'**il comprend en outre les étapes suivantes :
- analyse des données de mâchoire obtenues pour déterminer une exhaustivité des données de mâchoire et, en cas de données de mâchoire incomplètes et donc de lacunes dans les données de mâchoire :
- déplacement automatique respectif du ou des miroirs (30, 40) dans l'embout buccal (20) vers les positions de balayage correspondant aux lacunes,
- balayage supplémentaire d'un segment de l'arcade dentaire dans la bouche d'un repère d'étalonnage (50) présent sur l'enveloppe (90) de l'embout buccal (20) et correspondant à la position de balayage, et
- assemblage des clichés individuels, réalisés aux fins de la correction, avec les clichés individuels déjà disponibles.

18. Procédé selon la revendication 16 ou 17, **caractérisé en ce que** l'étalonnage présente les étapes suivantes :
- balayage unique ou multiple de la face interne de la mâchoire avec balayage simultané des repères d'étalonnage respectifs (50), tandis que la position de balayage est déviée uniquement au moyen du miroir central (30) sur la face interne de la mâchoire,
- rotation du miroir central (30) et ainsi balayage de l'ensemble de l'arcade dentaire conjointement avec les repères d'étalonnage (50) respectifs, depuis l'intérieur,
- assemblage des clichés individuels pour obtenir un cliché global de la face interne de la mâchoire, les contours de chacune des dents étant assemblés,
- analyse de la localisation des repères d'étalonnage (50) et comparaison avec les positions de consigne des localisations pour déterminer un écart,
- calcul d'une règle d'égalisation à partir dudit écart, pour savoir dans quelle mesure les repères d'étalonnage (50) mesurés doivent être égalisés, afin de les faire coïncider avec les repères d'étalonnage (50) réels, et
- utilisation de cette règle d'égalisation sur l'arcade dentaire mesurée afin de corriger les données de mâchoire obtenues.

19. Procédé selon la revendication 18, **caractérisé en ce que**, après l'étalonnage, les données suivantes pour les clichés de la face supérieure et de la face externe de l'arcade dentaire sont ajoutées à l'arcade dentaire mesurée.

20. Procédé selon l'une des revendications 16 à 19, **caractérisé en ce qu'**une face interne des dents est balayée en utilisant uniquement un miroir (30), qui se trouve centré au milieu de la cavité buccale et qui est incliné de manière à dévier le segment de balayage sur la face interne des dents.

21. Procédé selon l'une des revendications 16 à 20, **caractérisé en ce que**, lors du balayage des surfaces occlusales, la zone de balayage est déviée vers l'extérieur, par un miroir central (30), vers le deuxième miroir (40), lequel se trouve perpendiculairement au-dessus ou au-dessous des dents, et est ensuite déviée par le deuxième miroir (40) d'environ 90° sur la surface occlusale.

22. Procédé selon la revendication 21, **caractérisé en ce que**, lors du balayage de la face externe des dents, le deuxième miroir (40) est positionné davantage vers l'extérieur que lors du balayage des surfaces occlusales, puis la zone de balayage est déviée depuis l'extérieur vers la surface des dents.

23. Procédé selon l'une des revendications 16 à 22, **caractérisé en ce que**, lorsqu'un embout buccal (20) est transparent en haut et en bas, une inclinaison des miroirs (30, 40) occasionne un changement de balayage entre la mâchoire inférieure et la mâchoire supérieure.
